(19) **Europäisches Patentamt / European Patent Office / Office européen des brevets**

(11) **EP 4 512 832 A1**

(12) **EUROPEAN PATENT APPLICATION**
published in accordance with Art. 153(4) EPC

(43) Date of publication:
26.02.2025 Bulletin 2025/09

(21) Application number: 23791268.8

(22) Date of filing: 19.04.2023

(51) International Patent Classification (IPC):
*C08B 37/08* (2006.01)    *A61K 8/73* (2006.01)

(52) Cooperative Patent Classification (CPC):
A61K 8/73; A61K 31/728; A61P 17/00;
A61P 17/02; A61P 17/10; A61P 17/16; A61P 17/18;
A61P 31/04; C08B 37/003; Y02A 50/30

(86) International application number:
PCT/CN2023/089192

(87) International publication number:
WO 2023/202613 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.04.2022 CN 202210424292

(71) Applicants:
• Bloomage Biotechnology Corporation Limited
Jinan, Shandong 250101 (CN)
• Bloomage Biotech (Tianjin) Co., Ltd.
Tianjin 300270 (CN)

(72) Inventors:
• FENG, Ning
Jinan, Shandong 250101 (CN)

• ZONG, Wenbin
Jinan, Shandong 250101 (CN)
• SHI, Yanli
Jinan, Shandong 250101 (CN)
• WANG, Xiujuan
Jinan, Shandong 250101 (CN)
• ZHANG, Boxi
Jinan, Shandong 250101 (CN)
• WU, Jiajing
Jinan, Shandong 250101 (CN)
• LIU, Wenjun
Jinan, Shandong 250101 (CN)
• GUO, Xueping
Jinan, Shandong 250101 (CN)

(74) Representative: Abel & Imray LLP
Westpoint Building
James Street West
Bath BA1 2DA (GB)

(54) **PREPARATION METHOD AND APPLICATION OF ZINC HYALURONATE**

(57) The present application provides a preparation method for zinc hyaluronate, comprising the following steps: adding sodium hyaluronate into an acidic water-comprising organic medium that contains a zinc salt, and carrying out primary replacement to obtain a zinc hyaluronate precipitate; washing the precipitate using a washing liquid; dehydrating the washed precipitate using a dehydration liquid and drying same to obtain a zinc hyaluronate powder. The present application optimizes, on the basis of a solid replacement method, the zinc salt concentration in the replacement liquid, the replacement liquid volume, the replacement time, and the number of replacements, and solves the problems of numerous replacements, long time, large molecular weight consumption, and low zinc salt utilization rate in existing zinc hyaluronate production processes. The production cycle is greatly shortened, the replacement efficiency is improved, the contact time between the raw materials and the acidic replacement liquid is reduced, and the molecular weight of the product is prevented from being greatly lowered. The present application provides a method for rapidly and efficiently producing zinc hyaluronate, which is suitable for large-scale production of zinc hyaluronate.

EP 4 512 832 A1

Description

## TECHNICAL FIELD

The present application relates to a preparation method and application of zinc hyaluronate, which belongs to the technical field of hyaluronate.

## BACKGROUND

Hyaluronic acid (HA) is a polysaccharide composed of glucuronic acid and acetylglucosamine disaccharide units. The hyaluronic acid is widely used in the field of cosmetics due to its physiological functions such as moisturizing, nutrition, repair and damage prevention. Research has shown that in an aqueous solution with a pH value of 6.0 to 6.5, $Zn^{2+}$ can be combined with the oxygen-comprising donor groups in HA to form a zinc hyaluronate (Zn-HA) complex.

Typically, zinc hyaluronate is obtained by exchanging sodium ions in sodium hyaluronate with zinc ions in zinc salts.

A Hungarian patent reported the preparation process of powdered Zn-HA: the Na-HA was dissolved in water, followed by adding zinc salt, and the mixture was precipitated with an organic solvent, and then dehydrated and dried with an organic solvent. The ion exchange resin method currently used in China is: the sodium hyaluronate is dissolved and then loaded on an ion exchange column to convert Na-HA into Zn-HA, which is then precipitated with ethanol and dehydrated to obtain the Zn-HA. The common point between the above two methods is that the Na-HA is dissolved first and ion exchange is carried out in the solution. The former has a simpler route, but there is a higher sodium ion content in the product; the latter occupies more equipments and is more complicated to operate, but there is a higher zinc ion content in the product.

Patent CN100355790C discloses a method for preparing zinc hyaluronate, which provides a method for preparing powdered zinc hyaluronate, wherein the Na-HA solid is directly ion-exchanged with zinc salts in an alcohol solution to generate zinc hyaluronate.

Patent CN111647100A discloses a method for preparing zinc hyaluronate with high molecular weight, which obtains the best preparation conditions by exploring the preparation conditions such as the pH of the replacement solution, the number of replacements, the pH of the washes, the number of washes, the pH of the dehydration solution, and the number of dehydrations. The molecular weight of the obtained zinc hyaluronate may reach more than 1000 kDa, the light transmittance is greater than 99.5%, and the zinc content is greater than 7.0%.

The above processes all adopt multiple replacements, which have the problems of long cycle, large amount of organic solvent, low zinc salt utilization rate and large reduction in product molecular weight.

Zinc hyaluronate is the zinc salt of hyaluronic acid, which has the following efficacies except for the moisturizing properties of hyaluronic acid: anti-inflammatory, barrier repair, repair of skin damage, improvement of skin texture, removing black and whitening, etc., and can be used in the field of cosmetics. Furthermore, zinc hyaluronate also has physiological functions such as lubrication, antimicrobial effects, prevention and treatment of peptic ulcers, and promotion of wound repair, and is widely used in the medical field.

Patent CN102834417A discloses a method for preparing a hyaluronic acid metal salt, a method for preparing cosmetics comprising a hyaluronic acid metal salt, and a zinc hyaluronate and preparing method thereof, in which the skin was treated by a tape stripping method to destroy the barrier function of the skin, and essence water comprising 0.1% zinc hyaluronate was applied. From the experimental results, it can be seen that the zinc hyaluronate has an excellent effect of restoring the transepidermal water evaporation and can effectively repair the skin barrier. In addition, a effect on improving skin redness was also found in the zinc hyaluronate.

Patent CN112691049A discloses a shampoo composition comprising zinc hyaluronate. Zinc hyaluronate is mainly used to significantly inhibit Malassezia on the scalp, thereby playing the role of removing dandruff and relieving itching. In addition, an oil-controlling and soothing effect is achieved when the zinc hyaluronate is used in combination with other ingredients.

Patent CN102961396A discloses the use of hyaluronate in the manufacture of a medicament for treating skin diseases, drug combination and preparation method thereof, and provides a pharmaceutical composition (cream) comprising zinc hyaluronate and/or sodium hyaluronate, wherein the preparation with hyaluronate metal salt, especially zinc hyaluronate as the main active ingredient, has a definite effect on the treatment of skin barrier dysfunction, and can regulate the physiological function of the skin and repair the skin barrier function.

## SUMMARY

In view of the long-standing problems in the production of zinc hyaluronate, the present application optimizes the zinc salt concentration in the replacement solution, the volume of the replacement solution, the replacement time, and the number of replacements based on the solid replacement method, thereby greatly shortening the production cycle, improving the replacement efficiency, reducing the contact time between the raw material and the acidic replacement solution, and avoiding a significant reduction in the molecular weight of the product.

In view of the problems existing in the current preparation methods of zinc hyaluronate, the present application provides a method for producing zinc hyaluronate rapidly and efficiently, which is suitable for large-scale production of zinc hyaluronate.

Specifically, this application adopts the following technical solutions:

1. A method for preparing a zinc hyaluronate, comprising the following steps:

adding sodium hyaluronate to an acidic aqueous organic medium comprising zinc salt to perform one replacement to obtain a zinc hyaluronate precipitate.

2. According to the preparation method of item 1, wherein the mass ratio of the added amount of the sodium hyaluronate to the zinc salt is 1:0.5 to 1:3.5, preferably 1:1 to 1:3.

3. The method according to item 1 or 2, wherein the concentration of an organic solvent in the acidic aqueous organic medium comprising azinc salt is 55% tp 95% (v/v), preferably 55% to 70% (v/v), the concentration of zinc ion is 1% to 3% (w/v), and the pH of the acidic aqueous organic medium comprising a zinc salt is 5.0 to 6.9.

4. The method according to any one of items 1 to 3, wherein the molecular weight of the sodium hyaluronate is 1 kDa to 3000 kDa.

5. The method according to any one of items 1 to 4, wherein the replacement time is 1 to 24 hours.

6. The method according to any one of items 1 to 5, further comprising washing the zinc hyaluronate precipitate with a washing solution, then dehydrating the washed precipitate with a dehydrating solution, and drying the precipitate to obtain a zinc hyaluronate powder.

7. A zinc hyaluronate with low molecular weight prepared by the method described in any one of items 1 to 6, preferably, the molecular weight of the zinc hyaluronate with low molecular weight is less than or equal to 1000 kDa.

8. A zinc hyaluronate with high molecular weight prepared by the method according to any one of items 1 to 6, preferably, the molecular weight of the zinc hyaluronate with high molecular weight is greater than 1000 kDa.

9. A zinc hyaluronate, wherein the molecular weight of the zinc hyaluronate is 2 kDa to 2500 kDa, and the transmittance of the zinc hyaluronate in a 0.5wt% aqueous solution is $\geq$88.1%, preferably $\geq$ 99.1%.

10. Use of the zinc hyaluronate prepared by the method according to any one of items 1 to 8 or the zinc hyaluronate according to item 9 in skin moisturizing, skin oil control, inhibiting harmful bacteria on the skin, anti-oxidation, scar repair, inhibiting scar , and preventing skin wound infection,

> preferably, the inhibiting harmful bacteria on the skin comprises removing acne and dandruff;
> preferably, the preventing skin wound infection comprises preventing skin wound infection caused by intradermal and microneedle injection.

11. A method for skin moisturizing, skin oil control, inhibiting harmful bacteria on the skin, anti-oxidation, scar repair, inhibiting scar or preventing skin wound infection, comprising administering zinc hyaluronate prepared by the method according to any one of items 1 to 8 or the zinc hyaluronate according to item 9 to a subject,

> preferably, the inhibiting harmful bacteria on the skin comprises removing acne and dandruff;
> preferably, the preventing skin wound infection comprises preventing skin wound infection caused by intradermal and microneedle injection.

12. Use of the zinc hyaluronate prepared by the method according to any one of items 1 to 8 or the zinc hyaluronate according to item 9 in skin care products.

13. Use of a zinc hyaluronate in reducing skin oil content, preferably the zinc hyaluronate is the zinc hyaluronate with low molecular weight,
further preferably, the molecular weight of the zinc hyaluronate with low molecular weight is 2 kDa to 1000 kDa, preferably 5 kDa to 500 kDa.

14. Use of zinc hyaluronate in reducing porphyrin content in skin oil, preferably the zinc hyaluronate is a zinc hyaluronate with low molecular weight,
further preferably, the molecular weight of the zinc hyaluronate with low molecular weight is 2 kDa to 1000 kDa, preferably 5 kDa to 500 kDa.

15. Use of zinc hyaluronate in removing hydroxyl radicals from the skin surface, preferably the zinc hyaluronate is a zinc hyaluronate with low molecular weight,
further preferably, the molecular weight of the zinc hyaluronate with low molecular weight is 2 kDa to 1000 kDa, preferably 5 kDa to 500 kDa.

16. Use of the zinc hyaluronate in removing superoxide anion free radicals from the skin surface, preferably, wherein the zinc hyaluronate is a zinc hyaluronate with high molecular weight.
further preferably, the molecular weight of the zinc hyaluronate with high molecular weight is 1000 kDa to 2500 kDa, preferably 1100 kDa to 2000 kDa.

**Effects of the Invention**

1. Process improvement: the application optimizes the zinc salt concentration in the replacement solution, the volume of the replacement solution, the replacement time, and the number of replacements based on the solid replacement method, thereby greatly shortening the production cycle, improving the replacement efficiency, reducing the contact time between the raw material and the acidic replacement solution, and avoiding a significant reduction in the molecular weight of the product. The present application provides a method for producing zinc hyaluronate rapidly and efficiently, which is suitable for large-scale production of zinc hyaluronate.
2. Skin care effect: the zinc hyaluronate prepared in the application has the effects of moisturizing, inhibiting harmful bacteria on the skin, repairing damage, oil control, anti-oxidation, removing fine lines, etc., and can be used for skin care. Zinc hyaluronate is weakly acidic, and can ensure a weakly acidic environment for the skin (including the scalp). It may also inhibit the growth of some pathogenic microorganisms, protect the skin from invasion by various fungi, and repair inflammation and damage to the skin. In addition, zinc hyaluronate may maintain skin moisture, protect and enhance the natural protective barrier of the skin, therefore has a certain oil control effect in terms of regulating the secretion of oil by sebaceous glands, and maintaining water and oil balance. Therefore, zinc hyaluronate plays an important role in maintaining a healthy ecological environment of the skin (including the scalp).
3. The benefits of the zinc hyaluronate are gradually being discovered, but it is mostly studied and used in the medical field. Since there is no systematic and comprehensive study on the effects of zinc hyaluronate raw materials, therefore, this application conducts series of studies on the effects of zinc hyaluronate with different molecular weights, which has a more far-reaching significance for the application of zinc hyaluronate and the development of the industry.

**BRIEF DESCRIPTION OF THE DRAWINGS**

Figure 1A shows the effect of zinc hyaluronate I on water content in the stratum corneum;

Figure 1B shows the effect of zinc hyaluronate II on water content in the stratum corneum;

Figure 2A shows the effect of zinc hyaluronate I on skin oil content;

Figure 2B shows the effect of zinc hyaluronate II on skin oil content;

Figure 3A shows the effect of zinc hyaluronate I on the porphyrin content in the T region;

Figure 3B shows the effect of zinc hyaluronate II on the porphyrin content in the T region;

Figure 4 shows the scavenging effect of zinc hyaluronate on hydroxyl free radicals;

Figure 5 shows the scavenging effect of zinc hyaluronate on superoxide anion free radicals;

Figure 6A shows the changes in collagen I content;

Figure 6B shows the changes in collagen III content.

**DETAILED DESCRIPTION**

The present application is further described below in conjunction with examples. It should be understood that the examples are only used to further describe and illustrate the present application and are not used to limit the present application.

Unless otherwise defined, technical and scientific terms used in this description have the same meanings as commonly understood by those skilled in the art. Although methods and materials similar or equivalent to those described herein can be used in experiments or applications, the materials and methods are described below. In case of conflict, the present description, including definitions, will control and the materials, methods, and examples are illustrative only and not limiting. The present application is further described below in conjunction with specific embodiments, but is not intended to limit the scope of the present application.

The method for rapidly and efficiently producing zinc hyaluronate of the present application comprises the following steps:

preparing an acidic aqueous organic medium comprising a zinc salt;
soaking a sodium hyaluronate solid in the acidic aqueous organic medium comprising a zinc salt, stirring and replacing, and controlling the replacement time to make the zinc ion content reach the required level;
adding an acidic aqueous organic medium to the supernatant-removed residue after the zinc ion content reaches the requirement, then washing away the excess ions;
after washing, the product is dehydrated and then vacuum dried to obtain zinc hyaluronate.

In the present application, the zinc salt refers to a salt that can at least partially dissociate in an aqueous solution to generate zinc ions. Exemplary zinc salts include, but are not limited to, zinc lactate, zinc oxide, zinc chloride, zinc phosphate, zinc citrate, zinc acetate, zinc sulfate, zinc nitrate, zinc borate, zinc butyrate, zinc carbonate, zinc formate, zinc gluconate, zinc glycerate, zinc glycolate, zinc oxide, zinc phosphate, zinc picolinate, zinc propionate, zinc salicylate, zinc silicate, zinc stearate, zinc tartrate, zinc undecylenate, and mixtures thereof.

In certain preferred embodiments, the zinc salt is zinc chloride, zinc acetate, zinc sulfate, zinc nitrate or zinc lactate.

In the present application, the organic medium is an organic medium that has good compatibility with water but in which sodium hyaluronate or zinc hyaluronate is insoluble or slightly soluble, such as alcohol organic solvents, ketone organic solvents, an amide solvents or acetonitrile, preferably alcohol organic solvents or ketone organic solvents.

Exemplary alcohol organic solvents include, but are not limited to, methanol, ethanol, isopropanol, propanol, n-butanol, diacetone alcohol, ethylene glycol monoethyl ether, ethylene glycol monobutyl ether, propylene glycol butyl ether, propylene glycol monomethyl ether, and diethylene glycol monobutyl ether, and the like.

In certain preferred embodiments of the present application, the alcohol organic solvent is methanol and ethanol.

Exemplary ketone solvents include, but are not limited to, methyl ethyl ketone, methyl isobutyl ketone, 1-methyl-2-pyrrolidone, cyclohexanone, or acetone, and the like.

In certain preferred embodiments of the present application, the ketone organic solvent is acetone.

Exemplary amide solvents include, but are not limited to, N,N-dimethylacetamide, N,N-dimethylformamide, dimethyl sulfoxide, N-methylpyrrolidone, or formamide, and the like.

In the present application, the acidic aqueous organic medium refers to any organic medium as described above dissolved in water to form an aqueous organic medium of a certain proportion, and the pH value of the aqueous organic medium is adjusted to make it acidic. The substance for adjusting the pH value of the aqueous organic medium may be hydrochloric acid, glacial acetic acid, sulfuric acid, phosphoric acid, etc.

In certain preferred embodiments of the present application, the pH value of the acidic aqueous organic medium ranges from 5.0 to 6.9, for example, the pH value may be 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, or 6.9.

In an acidic environment, the sodium ion dissociation degree of sodium hyaluronate solid is high, making it easier to replace ions. However, if the pH is too low, sodium hyaluronate will be degraded, affecting the molecular weight of the product. The longer the product is in contact with the acidic aqueous medium, the more the molecular weight will decrease. Therefore, it is very important to control the appropriate pH and production cycle.

In certain preferred embodiments of the present application, the mass concentration of the organic medium is 55wt% to 95wt%, preferably 55wt% to 70wt%.

Within this concentration range, sodium hyaluronate is insoluble and in solid form. The lower the concentration of the aqueous medium, the higher the dispersion of sodium hyaluronate therein, but the sedimentation is slow, the loss is high, and the cycle is long; conversely, the dispersion is low, the loss is small, and the sedimentation is fast.

The concentration of zinc ions in the acidic aqueous organic medium comprising a zinc salt is 1wt% to 3wt%, for example, 1wt%, 1.1wt%, 1.2wt%, 1.3wt%, 1.4wt%, 1.5wt%, 1.6wt%, 1.7wt%, 1.8wt%, 1.9wt%, 2.0wt%, 2.1wt%, 2.2wt%, 2.3wt%, 2.4wt%, 2.5wt%, 2.6wt%, 2.7wt%, 2.8wt%, 2.9wt%, or 3wt%.

The concentration of zinc ions is related to the concentration of the organic medium in the acidic aqueous organic medium. The higher the concentration of the organic medium, the lower the solubility of zinc ions. Therefore, under the appropriate concentration of aqueous organic medium, the zinc ion concentration is preferably 1wt% to 3wt%, which may not only ensure that the zinc ion concentration reaches a certain level, but also promote the replacement reaction well and ensure that the zinc salt is completely dissolved in the acidic aqueous organic medium.

In the present application, the sodium hyaluronate is a white or off-white solid, and the molecular weight and uronic acid content of the sodium hyaluronate may be any value.

In a preferred embodiment of the present application, the molecular weight of the sodium hyaluronate is 1 kDa to 3000 kDa, preferably 10 kDa to 2500 kDa, and the uronic acid content is 40wt% to 50wt%, preferably 45wt% to 50wt%.

Due to the possibility of degradation during the preparation process, the molecular weight of the selected sodium hyaluronate is higher than the required molecular weight of zinc hyaluronate.

The ratio of the feed mass of sodium hyaluronate to the mass of the zinc salt is 1:0.5 to 1:3.5, for example, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, 1:2.0, 1:2.1, 1:2.2, 1:2.3, 1:2.4, 1:2.5, 1:2.6, 1:2.7, 1:2.8, 1:2.9, 1:3, 1:3.1, 1:3.2, 1:3.3, 1:3.4, or 1:3.5, preferably 1:1 to 1:3.

The time of replacement under stirring is 1 to 24 hours, preferably 5 to 16 hours.

Sodium hyaluronate is subjected to one replacement reaction in an acidic aqueous organic medium comprising a zinc salt. The higher the zinc ion concentration in the acidic aqueous organic medium, the larger the total amount of zinc salt used; and the longer the replacement time, the higher the zinc ion content in the product, that is, the higher the replacement rate. Depending on the content of zinc salt in the acidic aqueous medium, the volume of the replacement solution is controlled by controlling the total amount of zinc salt. When the ratio of the feed mass of sodium hyaluronate to the mass of zinc salt is 1: (0.5-3.5), the replacement efficiency is high. The longer the time of replacement under stirring, the higher the replacement efficiency, but the longer the time, the more the product degrades and the more the molecular weight decreases. Therefore, the replacement time is controlled within 1 to 24 hours, and further preferably, the replacement time is within 5 to 16 hours.

Furthermore, in the above preparation method, the pH of the acidic aqueous organic medium in the washing process is greater than or equal to 5.0 and less than 6.9, the concentration of the organic medium is 70wt% to 85wt%, and the number of washing times is 2 to 6 times.

Furthermore, after the zinc ions are replaced to meet the requirements, the product is washed to wash away excess unbound ions and make the bound ions more stable. The acidic aqueous organic medium is used for washing, in the acidic aqueous organic medium, the aqueous organic medium is an organic medium that has good compatibility with water but in which sodium hyaluronate or zinc hyaluronate is insoluble or slightly soluble, preferably an alcohol organic solvent or a ketone organic solvent, and among them, ethanol, methanol, and acetone, etc. are commonly used.

The concentration of the organic medium in the acidic aqueous organic medium is 70wt% to 85wt% and the pH is 5.0 to 6.9.

The standard for the washing process is based on the removal of all excess ions.

In order to improve the washing effect and reduce the generation of waste water, multiple washing, stirring and soaking may be carried out during washing process.

Furthermore, after washing is completed, the product is dehydrated using a neutral aqueous organic medium, wherein the concentration of the organic medium is greater than or equal to 90wt%; the organic medium is an organic medium that has good compatibility with water but in which sodium hyaluronate or zinc hyaluronate is insoluble or slightly soluble, preferably an alcohol organic solvent or a ketone organic solvent, and among them, ethanol, methanol, and acetone, etc. are commonly used.

After dehydration, the aqueous organic medium in the supernatant is removed and the precipitate is dried to obtain the zinc hyaluronate solid.

The drying method is vacuum drying, and the drying temperature may be adjusted depending on the molecular weight requirements and drying loss requirements of the product, generally ranging from 20°C to 75°C.

Furthermore, the zinc ion content of the zinc hyaluronate obtained by the above preparation method is 6.0wt% to 9.0wt%, the replacement rate is 75% to 100%, and the other indicators are: pH 5.5 to 7.5; drying loss $\leq$ 15.0%, transmittance $\geq$ 99.0%, heavy metal $\leq$ 20ppm, protein $\leq$ 0.1%, zinc hyaluronate content is more than 90wt%, and zinc hyaluronate yield is more than 90%.

Furthermore, in the preparation process of zinc hyaluronate, in addition to the steps of replacement under stirring, washing, dehydrating and drying, a step of degrading sodium hyaluronate may also be included, and the degradation step is performed before replacement under stirring.

The degradation of sodium hyaluronate may be achieved by any method disclosed in the prior art, such as enzymatic hydrolysis, alkaline hydrolysis, etc.

Since sodium hyaluronate will degrade in an acidic environment, in a specific embodiment of the present application, when preparing zinc hyaluronate with lower molecular weight, sodium hyaluronate is first degraded in an acidic aqueous organic medium to degrade the molecular weight of sodium hyaluronate to a desired molecular weight, and then the degraded sodium hyaluronate is stirred and replaced to prepare zinc hyaluronate.

The pH for degradation is different from the pH for replacement under stirring. The pH of degradation is less than 5, preferably the pH is greater than 1 and less than 5.

After the degradation is completed, the sodium hyaluronate in which the pH has been adjusted with sodium hydroxide is added to the acidic aqueous organic medium comprising a zinc salt for replacement under stirring.

In the present application, the preparation method of zinc hyaluronate is further optimized after multiple experimental attempts based on the existing preparation method of zinc hyaluronate. During the preparation process, the mass ratio of sodium hyaluronate to zinc salt in the replacement process is adjusted to an appropriate ratio, such that the sodium hyaluronate and the zinc salt may achieve a good replacement effect through only one replacement, avoiding the need to repeatedly add zinc salt for multiple replacements in the prior art, which consumes manpower and material resources. The present application further optimizes the concentration of the solvent used in the preparation process and the molecular weight of sodium hyaluronate, strengthens the process parameters, and further guarantees the quality of the prepared zinc hyaluronate. Furthermore, the method for preparing zinc hyaluronate of the present application simplifies the preparation steps, saves resources, and the prepared zinc hyaluronate has good effect, and has obvious advantages in light transmittance and yield.

By regulating the degradation steps and selecting the molecular weight of sodium hyaluronate, zinc hyaluronate with different molecular weights within the range of 1 kDa to 2000 kDa may be obtained, for example, the molecular weight is 1kDa to 5kDa, 5kDa to 10kDa, 10kDa to 200kDa, 200kDa to 500kDa, 500kDa to 1000kDa, or 1000kDa to 2000kDa.

Furthermore, the zinc hyaluronate prepared in the present application has the functions of moisturizing, inhibiting harmful bacteria on the skin, repairing damage, oil control, anti-oxidation, removing fine lines, etc., maintaining a healthy skin (including scalp) ecological environment, and may be used as a raw material in the cosmetics field.

Zinc hyaluronate is a zinc salt of hyaluronic acid, which not only has the excellent properties of HA, but also has unique physiological functions and effects. Zinc hyaluronate with different molecular weights has different biological activities. Among them, zinc hyaluronate with low molecular weight, also called zinc hydrolyzed hyaluronate, refers to zinc hyaluronate with a molecular weight lower than 1000 kDa, which is obtained by the replacement reaction of sodium hydrolyzed hyaluronate and zinc ions. Experimental verification has shown that zinc hyaluronate has a significant inhibitory effect on a variety of skin surface bacteria (such as *Staphylococcus epidermidis, Malassezia, Propionibacterium acnes,* etc.), and the zinc hyaluronate with high molecular weight is more effective than zinc hydrolyzed hyaluronate. Zinc hyaluronate also has the effect of reducing the oil content in the skin epidermis. Porphyrin is a product of microbial metabolism and there is a certain correlation between the porphyrin content and the oil content in the skin. In areas with higher oil content, microbial metabolic activities are usually more vigorous, and the amount of porphyrin produced is higher than that of other areas. Zinc hyaluronate may reduce the porphyrin content in the T region to a certain extent, and the effect of zinc hyaluronate with low molecular weight is more significant. In addition, the zinc hyaluronate with low molecular weight may also reduce fine lines, improve skin texture and delay skin aging. In addition, zinc hyaluronate may significantly inhibit the synthesis of Collagen I, thereby affecting the expression ratio of Collagen I and Collagen III proteins and inhibiting the occurrence of scars caused by excessive expression of Collagen I.

Furthermore, the zinc hyaluronate may be used to prepare skin care products. The skin care is moisturizing, antibacterial, anti-inflammatory, oil control, anti-aging, and repair. Furthermore, the skin care products are toner, essence, gel, lotion, cream, facial mask, makeup, soap, facial cleanser, shampoo, conditioner and shower gel. Furthermore, skin care products of various dosage forms comprising zinc hyaluronate are all protected by the application.

The zinc hyaluronate prepared in the present application is used in skin moisturizing, skin oil control, inhibiting harmful bacteria on the skin, anti-oxidation, scar repair, inhibiting, and preventing skin wound infection.

Through a large number of experimental studies, the present application has found that there is a certain correlation between the porphyrin content and the oil content in the skin. In areas with higher oil content, microbial metabolic activities are usually more vigorous, and the amount of porphyrin produced is higher than that of other areas. The zinc hyaluronate prepared in the present application may significantly maintain the skin porphyrin content at a lower level, thereby effectively controlling the skin oil content. In a preferred embodiment, the molecular weight of the zinc hyaluronate is 2 kDa to 1000 kDa, which has a better oil control effect, for example, the molecular weight is 2 kDa, 5 kDa, 10 kDa, 20 kDa, 30 kDa, 40 kDa, 50 kDa, 60 kDa, 70 kDa, 80 kDa, 90 kDa, 100 kDa, 150 kDa, 200 kDa, 250 kDa, 300 kDa, 350 kDa, 350 kDa, 400 kDa, 450 kDa, 500 kDa, 550 kDa, 600 kDa, 650 kDa, 700 kDa, 750 kDa, 800 kDa, 850 kDa, 900 kDa, 950 kDa, or 1000 kDa, and further preferably 5 kDa to 500 kDa.

The zinc hyaluronate prepared in the present application has a good effect in removing acne and dandruff; further research in the present application found that the zinc hyaluronate has a high inhibition rate on *Staphylococcus epidermidis,* which may reach about 50%, and the inhibition rate on *Malassezia furfur* may reach more than 70%. *Malassezia furfur* mainly causes dandruff and scurf problems, therefore the zinc hyaluronate in the present application has good use in removing dandruff; the inhibition rate of the zinc hyaluronate on *Propionibacterium acnes* is also more than 60%, and *Propionibacterium acnes* mainly causes skin acne problems. Therefore, the zinc hyaluronate of the present application also has good use in acne removal. Furthermore, the present application may also prevent skin wound infection caused by intradermal, microneedle injections, etc.

The hyaluronic acid prepared in the present application has good use in anti-oxidation. The zinc hyaluronate has a significant effect on the scavenging of hydroxyl radicals, and the hydroxyl radical scavenging rate of zinc hyaluronate with low molecular weight is higher, which can reach more than 80%. In a preferred embodiment, the effect is better when the molecular weight of the zinc hyaluronate with low molecular weight is 2 kDa to 1000 kDa, for example, the molecular weight is 2kDa, 5kDa, 10kDa, 20kDa, 30kDa, 40kDa, 50kDa, 60kDa, 70kDa, 80kDa, 90kDa, 100kDa, 150kDa, 200kDa, 250kDa, 300kDa, 350kDa, 350kDa, 400kDa, 450kDa, 500kDa, 550kDa, 600kDa, 650kDa, 700kDa, 750kDa, 800kDa, 850kDa, 900kDa, 950kDa, or 1000kDa, and further preferably 5kDa to 500kDa.

The present application further found that the zinc hyaluronate is very useful in removing superoxide anion free radicals from the skin surface. The zinc hyaluronate has a significant effect on removing superoxide anion free radicals, among which zinc hyaluronate with high molecular weight has higher removal ability, and the removal rate may reach more than 70%. In a preferred embodiment, the molecular weight of the zinc hyaluronate with high molecular weight is 1000 kDa to 2500 kDa, for example, the molecular weight is 1100 kDa, 1150 kDa, 1200 kDa, 1250 kDa, 1300 kDa, 1350 kDa, 1400 kDa, 1450 kDa, 1500 kDa, 1550 kDa, 1600 kDa, 1650 kDa, 170 0kDa, 1750 kDa, 1800 kDa, 1850 kDa, 1900 kDa, 1950 kDa, 2000 kDa, 2050 kDa, 2100 kDa, 2150 kDa, 2200 kDa, 2250 kDa, 2300 kDa, 2350 kDa, 2400 kDa, 2450 kDa, or 2500 kDa, and further preferably 1100 kDa to 2000 kDa.

Beneficial Effects

1. In the present application, the preparation of zinc hyaluronate can be achieved by only one replacement, and the zinc ion content of the prepared zinc hyaluronate is greater than 4wt%, the replacement rate is greater than 56%, the transmittance is greater than 88.0%, and the yield is more than 89%.

2. The preparation of the zinc hyaluronate of the present application is fast and efficient, which saves a lot of production time and manpower and material costs. The obtained zinc hyaluronate has a high yield and is suitable for large-scale industrial production.

Example

The following examples will be used to provide a detailed explanation of the present application. However, it should be understood that the present application can be implemented in various forms and should not be limited by the embodiments described herein. On the contrary, these embodiments are provided to enable a more thorough understanding of the present application and to fully convey the scope of the present application to those skilled in the art. The numerical ranges listed in this application include the data of the two endpoints of the numerical range, as well as each specific value in the numerical range, and the value can be combined with any endpoint to form a new small range.

In the following examples and comparative examples, the sodium hyaluronate raw materials used were all from Bloomage Biotechnology Co., Ltd.

In the following examples, the uronic acid content was detected by sulfuric acid-carbazole colorimetry, the sodium content and zinc content were detected by atomic absorption spectrophotometry, and the molecular weight was detected by intrinsic viscosity method.

In the following examples, the replacement rate refers to the replacement efficiency of zinc ions in zinc hyaluronate, and the replacement rate was calculated as follows: the ratio of the actual detected value of zinc ions in zinc hyaluronate to the theoretical value of zinc ions after complete replacement.

In the following examples, the yield is the ratio of the output amount to the feed amount, and the calculation formula is:

$$\text{Yield} = \text{output amount of zinc hyaluronate} / \text{feed amount of sodium hyaluronate} \times 100\%$$

In the following examples, the transmittance of a 0.5% aqueous solution was measured at 550 nm using a spectrophotometer.

In the following examples, unless otherwise specified, the concentrations are all in mass percentage.

Example 1

Reagents: ethanol, Bloomage Biotech Co., Ltd.;
Glacial acetic acid, Sinopharm Chemical Reagent Co., Ltd.;
Sodium hyaluronate, Bloomage Biotech Co., Ltd.;
Zinc acetate, Sinopharm Chemical Reagent Co., Ltd.;
Zinc chloride, Sinopharm Chemical Reagent Co., Ltd.;
Zinc sulfate, Sinopharm Chemical Reagent Co., Ltd.

170 L of a zinc acetate-ethanol solution comprising 1.88wt% zinc ions was prepared, wherein the ethanol concentration was about 55wt%, and the pH was adjusted to 6.5 with glacial acetic acid.

30 kg of sodium hyaluronate (molecular weight is 2500 kDa) solid was accurately weighted, which was then added to the above replacement solution, and replacement was carried out under stirring for 8 hours.

The mixture was allowed to stand until the supernatant became clear, the supernatant was removed, and 800 L of 70wt% ethanol aqueous solution (pH 6.3) was added for washing. The mixture was washed and stirred for 3 hours, and then allowed to stand until the supernatant became clear, the supernatant was removed, and 800 L of 70wt% ethanol aqueous solution (pH 6.3) was added for washing in the same manner. A total of 4 washes were performed.

After the last wash, the mixture was allowed to stand and the supernatant was removed, and 800 L of 90wt% ethanol solution was added for dehydration. Dehydration was performed twice.

Then it was transferred to a three-in-one dryer for vacuum drying at a drying temperature of 45°C, a vacuum degree of 0.10 MPa for 18 hours, and 28.33 kg of zinc hyaluronate was obtained.

Examples 2-17

The zinc hyaluronate of Examples 2-17 was prepared according to the method of Example 1. The amounts of various substances and technical parameters in the method are shown in the table. The technical parameters not mentioned in the table are the same as those in Example 1.

**Table 1 Contents of substances and technical parameters in each example**

| | Volume of replacement solution (L) | Zinc ion content in | Ethanol concentration in | pH of replacem | NaHA mass (kg) | NaHA : zinc salt | NaHA molecular weight | Types of Zinc Salts |
|---|---|---|---|---|---|---|---|---|
| example 1 | 170 | 1.88 | 55 | 6.5 | 30 | 1:0.3 | 2500k | Zinc Acetate |
| example 2 | 240 | 1.88 | 55 | 6.5 | 30 | 1:0.5 | 2500k | Zinc Acetate |
| example3 | 480 | 1.88 | 55 | 6.5 | 30 | 1:1 | 2500k | Zinc Acetate |
| example4 | 960 | 1.88 | 55 | 6.5 | 30 | 1:2 | 2500k | Zinc Acetate |
| example5 | 1440 | 1.88 | 55 | 6.5 | 30 | 1:3 | 2500k | Zinc Acetate |
| example6 | 1680 | 1.88 | 55 | 6.5 | 30 | 1:3.5 | 2500k | Zinc Acetate |
| example7 | 1920 | 1.88 | 55 | 6.5 | 30 | 1:4 | 2500k | Zinc Acetate |
| example8 | 480 | 1.88 | 70 | 6.5 | 30 | 1:1 | 500k | Zinc Acetate |
| example9 | 480 | 1.88 | 70 | 6.0 | 30 | 1:1 | 1730k | Zinc Chloride |
| example10 | 1330 | 0.91 | 75 | 5.7 | 30 | 1:1 | 50k | zinc sulfate |
| example 11 | 2130 | 0.5 | 85 | 5.1 | 30 | 1:1 | 1k | Zinc Chloride |
| example12 | 2130 | 0.5 | 60 | 6.2 | 30 | 1:1 | 2500k | Zinc Acetate |
| example13 | 1060 | 1 | 60 | 6.3 | 30 | 1:1 | 2500k | Zinc Acetate |
| example14 | 710 | 1.5 | 60 | 6.3 | 30 | 1:1 | 2500k | Zinc Acetate |

(continued)

|  | Volume of replacement solution (L) | Zinc ion content in | Ethanol concentration in | pH of replacem | NaHA mass (kg) | NaHA : zinc salt | NaHA molecular weight | Types of Zinc Salts |
|---|---|---|---|---|---|---|---|---|
| example15 | 570 | 1.88 | 60 | 6.3 | 30 | 1:1 | 2500k | Zinc Acetate |
| example16 | 480 | 2.2 | 60 | 6.3 | 30 | 1:1 | 2500k | Zinc Acetate |
| example17 | 350 | 3 | 60 | 6.3 | 30 | 1:1 | 2500k | Zinc Acetate |
| example18 | 300 | 3.5 | 60 | 6.3 | 30 | 1:1 | 2500k | Zinc Acetate |

Test Example 1 Product Performance and Quality of Zinc Hyaluronate

The product performance and quality of the zinc hyaluronate prepared in Examples 1-18 were measured, and the indicators are shown in Table 2. As can be seen from the table, the preparation of zinc hyaluronate can be achieved by only one replacement in the present application by adjusting the reasonable ratio and concentration of each component as well as appropriate parameter indicators, and the prepared zinc hyaluronate has the zinc ion content of greater than 4wt%, the replacement rate of greater than 56%, the transmittance of greater than 88.0%, the yield of more than 89%, and the quality indicators of the product meet the requirements.

**Table 2 Product Performance Indicators of Zinc Hyaluronate**

|  | zinc content | sodium content | replacement rate | ZnHA molecular weight | yield | transmittance | weight loss caused by drying | pH | properties |
|---|---|---|---|---|---|---|---|---|---|
| example1 | 4.45 | 2.21% | 56.26% | 1950k | 89.10% | 99.50% | 7.8 | 6.3 | white powder |
| example 2 | 6.81 | 0.95% | 86.09% | 1920k | 92.70% | 99.30% | 8.6 | 6.5 | white powder |
| example3 | 7.59 | 0.58% | 95.95% | 1960k | 93.50% | 99.40% | 6.9 | 6.2 | white powder |
| example4 | 7.88 | 0.55% | 99.62% | 1910k | 92.20% | 99.50% | 7.5 | 6.7 | white powder |
| example5 | 7.91 | 0.53% | 99.98% | 1860k | 93.40% | 99.30% | 8.9 | 6.3 | white powder |
| example6 | 7.93 | 0.50% | 100.24% | 1800k | 93.80% | 99.10% | 8.4 | 6.4 | white powder |
| example7 | 7.94 | 0.46% | 100.37% | 1820k | 93.10% | 96.50% | 9.1 | 6.5 | white powder |
| example8 | 7.51 | 0.60% | 94.94% | 390k | 93.10% | 99.40% | 7.9 | 6.2 | white powder |
| example9 | 6.86 | 0.82% | 86.73% | 1270k | 93.50% | 99.30% | 8 | 6.4 | white powder |
| example 10 | 7.02 | 0.78% | 88.75% | 45k | 93.20% | 99.50% | 7.4 | 6.3 | white powder |
| example11 | 5.99 | 1.23% | 75.73% | 1k | 90.20% | 99.40% | 7.2 | 6.2 | white pow-der |

# EP 4 512 832 A1

(continued)

| | zinc content | sodium content | replacement rate | ZnHA molecular weight | yield | transmittance | weight loss caused by drying | pH | properties |
|---|---|---|---|---|---|---|---|---|---|
| example 12 | 5.91 | 1.31% | 74.72% | 1810k | 92.70% | 99.40% | 7.9 | 6.6 | white powder |
| example 13 | 6.92 | 0.85% | 87.48% | 1840k | 92.70% | 99.40% | 8.6 | 6.5 | white powder |
| example 14 | 7.23 | 0.71% | 91.40% | 1880k | 92.70% | 99.50% | 6.7 | 6.2 | white powder |
| example 15 | 7.54 | 0.65% | 95.32% | 1910k | 93.90% | 99.30% | 7.5 | 6.7 | white powder |
| example 16 | 7.87 | 0.53% | 99.49% | 1650k | 92.70% | 99.20% | 8.1 | 6.3 | white powder |
| example 17 | 7.91 | 0.52% | 100.00% | 1920k | 92.70% | 99.20% | 8.4 | 6.4 | white powder |
| example 18 | 8.90 | 0.43% | 112.5% | 1900k | 92.60% | 88.1% | 9.2 | 6.5 | white powder |

Test Example 2 Study on the properties of zinc hyaluronate

Experimental Materials:

Zinc hyaluronate (Example 9, molecular weight is 1270kD), hyaluronate sodium (Bloomage Biotech Co., Ltd., molecular weight is 1230 kD)

Experimental process:

Aqueous solutions of 0.5% zinc hyaluronate and 0.5% sodium hyaluronate (comprising 0.13 g zinc acetate dihydrate) were prepared to ensure that the zinc ion content in both samples was 0.039%. The samples were named HA-Zn and HA-Na+Zn salt, respectively. The dynamic viscosity, transmittance and osmotic pressure of the two solutions were tested.

Experimental results:

(1) Dynamic viscosity

The dynamic viscosity of the sample solution with a concentration of 0.5% was measured at 25°C. The results are shown in Table 3.

Table 3

| Sample name | HA-Zn | HA-Na+Zn salt |
|---|---|---|
| dynamic viscosity (mPa·s) | 310.0 | 260.0 |

(2)Transmittance

The transmittance of the samples was measured at 550 nm using a spectrophotometer. The results are shown in Table 4.

**11**

Table 4

| Sample name | HA-Zn | HA-Na+Zn salt |
|---|---|---|
| transmittance (%) | 94.62% | 81.26% |

(3) Osmotic pressure

The osmotic pressure of the sample was measured with the osmotic pressure of water being 0. The results are shown in Table 5.

Table 5

| Sample name | HA-Zn | HA- Na +Zn salt |
|---|---|---|
| osmotic pressure (mOsm /kg) | +10 | +29 |

It can be seen from the above results that, compared with the sodium hyaluronate solution comprising the same zinc ion content, the dynamic viscosity and transmittance of zinc hyaluronate are higher, while the osmotic pressure is lower.

Test Example 2.1 Patch test

Experimental Materials

Sample 1: zinc hyaluronate with high molecular weight (Example 9, 1270 kD);
Sample 2: zinc hyaluronate with low molecular weight (Example 10, 45 kD)

Experimental process:

(1) Sample preparation

Purified water was used as a control, and sample 1 and sample 2 were prepared with purified water to a concentration of 1.0% and 0.5%, respectively.

(2) Skin patch test

The packaging of the patch tester was open, and 0.025mL of each prepared sample was measured and added into the small chamber. The patch tester was applied to the curved side of the forearm of the subject, and lightly pressed with the palm of the hand to make it evenly applied to the skin for 24 hours. There were 30 subjects.

3. Results Analysis

After removing the patch tester, observing and recording the reaction results according to Table 6 at 30 minutes, 24 hours, and 48 hours, respectively.

Table 6 Skin reaction grading standards for skin occlusive patch test

| Extent of reaction | Score | Skin reactions |
|---|---|---|
| - | 0 | negative reaction |
| ± | 1 | suspicious reaction, only weak erythema |
| + | 2 | weak positive reaction (erythema reaction); redness, infiltration, edema, and possible papules |
| ++ | 3 | strong positive reaction (herpes response); redness, infiltration, edema, papules, herpes; the reaction may exceed the test area |

(continued)

| Extent of reaction | Score | Skin reactions |
|---|---|---|
| +++ | 4 | extremely strong positive reaction (fusion herpes response); obvious erythema, severe infiltration, edema, and fusion herpes; reaction exceeds the test area |

Note: Refer to the "Technical Descriptions for Safety of Cosmetics" - 2015 edition.

Table 7 Patch test results statistics (total number of cases: 30)

| / | sample 1 | | sample 2 | | control |
|---|---|---|---|---|---|
| grade | 1.0%zinc hyaluronate (example 9) | 0.5%zinc hyaluronate (example 9) | 1.0%zinc hyaluronate (example 10) | 0.5%zinc hyaluronate (example 10) | purified water |
| grade 0 | 30 | 30 | 30 | 30 | 30 |
| grade 1 | 0 | 0 | 0 | 0 | 0 |
| grade 2 | 0 | 0 | 0 | 0 | 0 |
| grade 3 | 0 | 0 | 0 | 0 | 0 |
| grade 4 | 0 | 0 | 0 | 0 | 0 |

The results are shown in Table 7. All 30 subjects tested were negative for 1.0% and 0.5% zinc hyaluronate with high molecular weight and the zinc hyaluronate with low molecular weight, indicating no potential adverse reactions to the human body.

Test Example 2.2 Moisturizing Effect

Experimental Materials:

Emulsion comprising 0.5% of zinc hyaluronate I (Example 9); emulsion comprising 0.5% of zinc hyaluronate II (Example 10); blank emulsion (without zinc hyaluronate)

The other ingredients in the emulsion formula are shown in Table 8 below:

Table 8

| INCI | content% |
|---|---|
| CETEARYL GLUCOSIDE | 1.8 |
| BEHENYL ALCOHOL | 1.5 |
| CAPRYLIC/CAPRIC TRIGLYCERIDE | 3.0 |
| ETHYLHEXYL PALMITATE | 2.0 |
| DIMETHICONE | 2.0 |
| TREHALOSE | 0.5 |
| GLYCERIN | 3.0 |
| BUTYLENE GLYCOL | 3.0 |
| Zinc hyaluronate | 0.5 |
| XANTHAN GUM | 0.2 |
| HYDROXYACETOPHENONE | 0.4 |
| 1,2-HEXANEDIOL | 0.4 |
| Water (AQUA) | To 100 |

Experimental process:

The half-face comparison method was used, and the subjects were divided into two groups, A and B, with 10 subjects in each group;
Group A: The left face was treated with an emulsion comprising the zinc hyaluronate with high molecular weight (Example 9), and the right face was treated with a blank emulsion; Group B: The left face was treated with an emulsion comprising the zinc hyaluronate with low molecular weight (Example 10), and the right face was treated with a blank emulsion; the water content in the stratum corneum of the cheekbone region of the subjects' faces was measured before and after use.

Experimental results:

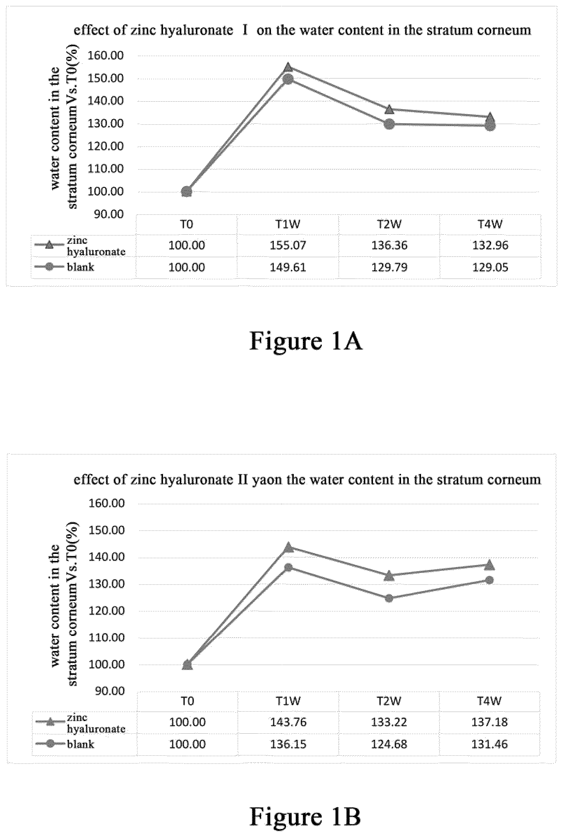

The results of Group A and Group B are shown in Figures 1A and 1B, respectively, and the initial value before use was set as 100%. Compared with the blank group, both the zinc hyaluronate with high molecular weight and the zinc hyaluronate with low molecular weight can increase the water content in the stratum corneum (4% to 8%) within 4 weeks of use.

Test Example 2.3: Oil control effect

Experimental Materials:

Two emulsions prepared in Test Example 2.2

Experimental process:

The half-face comparison method was used, and the subjects were divided into two groups, A and B, with 10 subjects in each group;
Group A: The left face was treated with an emulsion comprising the zinc hyaluronate with high molecular weight (Example 9), and the right face was treated with a blank emulsion; Group B: The left face was treated with an emulsion comprising the zinc hyaluronate with low molecular weight (Example 10), and the right face was treated with a blank emulsion; the oil secretion of the forehead, porphyrin content in the T region, and texture area of the facial cheekbone area of the subjects were tested before and after use.

Experimental results:

1. Effect of test samples on skin oil content

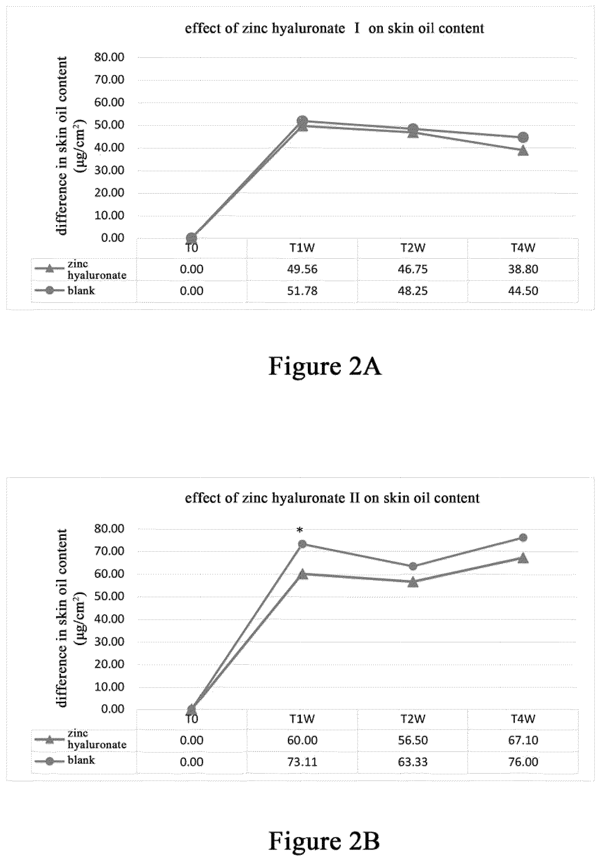

The results of Group A and Group B are shown in Figures 2A and 2B, respectively. The results are expressed as the difference in skin oil content compared with that before use. In Figure 2A, compared with the blank group, the zinc hyaluronate with high molecular weight group had a slightly lower skin oil content, and the average oil content decreased by 5.70 $\mu$g/cm$^2$ at the 4th week; while in Figure 2B, the zinc hyaluronate with low molecular weight group had a more significant effect in reducing oil, especially after 1 week of use, the average skin oil content was reduced by 13.11 $\mu$g/cm$^2$ compared with that of the blank group.

2. Effect of test samples on the porphyrin content in the T region

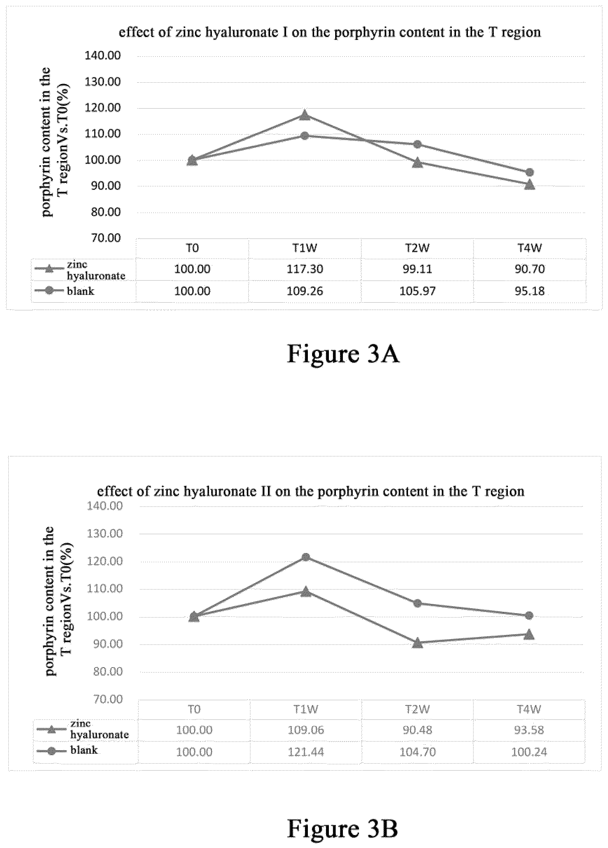

Porphyrin is a product of microbial metabolism. There is a certain correlation between the porphyrin content and the oil content in the skin. In areas with higher oil content, microbial metabolic activities are usually more vigorous, and the amount of porphyrin produced is higher than that of other areas. The results of Group A and Group B are shown in Figures 3A and 3B, respectively, and the initial value before use was set as 100%. In Figure 3A, compared with the blank group, zinc hyaluronate I was able to reduce the porphyrin content in the T region to a certain extent after 2 weeks of use (reduced by 4% to 6%); in Figure 3B, compared with the blank group, zinc hyaluronate II maintained a low porphyrin content in the T region (reduced by 7% to 14%) within 4 weeks of use.

Test Example 2.4: inhibiting harmful bacteria on the skin

Experimental Materials:

Zinc hyaluronate sample (Example 9, 1270 kD)

Experimental process:

1) Diluting the test bacterial suspension appropriately with PBS solution. The required concentration is: taking 0.1 mL and dropping it into 5.0 mL of control sample solution (PBS, phosphate buffer solution). The number of recovered bacteria is $1\times10^4$ to $9\times10^4$ cfu/mL.

2) Diluting the test sample with sterile standard hard water to the specified concentration.

3) Taking 5.0mL of the original or diluted test sample and placing it in a sterilized test tube. Keep the temperature at 20°C for 5 minutes.

4) Taking 0.1mL of the test bacterial solution and adding it to a test tube comprising 5.0mL of the sample. Mixing quickly and start timing immediately..

5) After the set time, taking 0.5 mL of the test bacteria and sample mixture, adding it to a test tube comprising 4.5 mL of sterilized PBS, and mixing thoroughly.

6) After standing for 10 minutes, taking 1 mL of the sample solution (or after appropriate dilution, taking 2 to 3 dilutions) and placing it in a sterile plate. Inoculating two sterile plates for each sample solution or dilution. Pouring 15 mL of nutrient agar medium (bacteria) or Sabouraud agar medium (candida albicans) cooled to 40°C to 45°C, rotating the plate to make it fully uniform, turning the plate over after the agar solidifies, and culturing at $(35\pm2)$°C for 48 hours (bacteria) or 72 hours (candida albicans), and then counting the live bacterial colonies.

7) Substituting PBS for the test sample and following the above steps as the control sample.

8) Calculating the antibacterial rate

**Antibacterial rate(%)= (average colony count of control sample - average colony count of test sample)/ (average colony count of control sample) $\times$100%**

Table 9

| Experimental strain | Action time /h | Action concentration | Antibacterial rate % | Effect evaluation criteria (antibacterial rate %) | |
|---|---|---|---|---|---|
| | | | | 90% | 50%~90% |
| Staphylococcus epidermidis | 2 | 0.5% | 22.06 | The product has strong antibacteria 1 effect | The product has antibacterial effect |
| | 4 | 0.5% | 29.59 | | |
| | 8 | 0.5% | 50.03 | | |
| Malassezia furfur | 2 | 0.5% | 42.86 | | |
| | 4 | 0.5% | 62.70 | | |
| | 8 | 0.5% | 72.06 | | |
| Propionibacterium acnes | 2 | 0.5% | 44.04 | | |
| | 4 | 0.5% | 53.77 | | |
| | 8 | 0.5% | 62.10 | | |

The results are shown in Table 9: after 8 hours of treatment with 0.5% zinc hyaluronate solution, the inhibition rate against Staphylococcus epidermidis was 50.03%, the inhibition rate against Malassezia furfur was 72.06%, and the inhibition rate against Propionibacterium acnes was 62.10%. Therefore, zinc hyaluronate has an inhibitory effect on harmful bacteria on the skin surface and can prevent skin wound infection caused by intradermal, and microneedle injections, etc.; it can also be used in products for removing dandruff (caused by Malassezia furfur) and removing acne (caused by Propionibacterium acnes).

Test Example 2.5 Antioxidant Effect

(1) Hydroxyl radical scavenging effect

Experimental materials: 0.5% zinc hyaluronate I (Example 9, 1270 kD) solution sample, 0.5% zinc hyaluronate II (Example 10, 45 kD) sample; 0.5% sodium hyaluronate solution sample (Bloomage Biotechnology Co., Ltd., 1230 kD), 0.5% zinc acetate (dihydrate) solution;

Experimental methods:

The salicylic acid method was used to determine the hydroxyl radical scavenging ability of zinc hyaluronate samples. A certain amount of $H_2O_2$ and $FeSO_4$ were mixed to produce hydroxyl radicals by Fenton reaction. Adding salicylic acid to the reaction system can capture hydroxyl radicals and produce 3-hydroxysalicylic acid and 5-hydroxysalicylic acid. Both products have strong absorption at 510nm. The reaction equation is as follows:

$$H_2O_2+Fe^{2+} \rightarrow OH+OH^-+Fe^{3+}$$

The addition of the sample will compete with salicylic acid for the reaction with OH, thereby reducing the production of 3-hydroxysalicylic acid and 5-hydroxysalicylic acid. The fixed reaction time method was used to measure the absorbance of the reaction solution at 510 nm and compare it with the blank solution to determine the scavenging effect of the analyte on hydroxyl free radicals.
The scavenging rate is calculated as follows:

$$S/\%=A_0-(A_x-A_{x0})/A_0\times100$$

In the formula, S is the scavenging rate, $A_0$ is the absorbance value of the blank control, $A_x$ is the absorbance value when the sample is added, and $A_{x0}$ is the absorbance value without adding the color developer.

Experimental results:

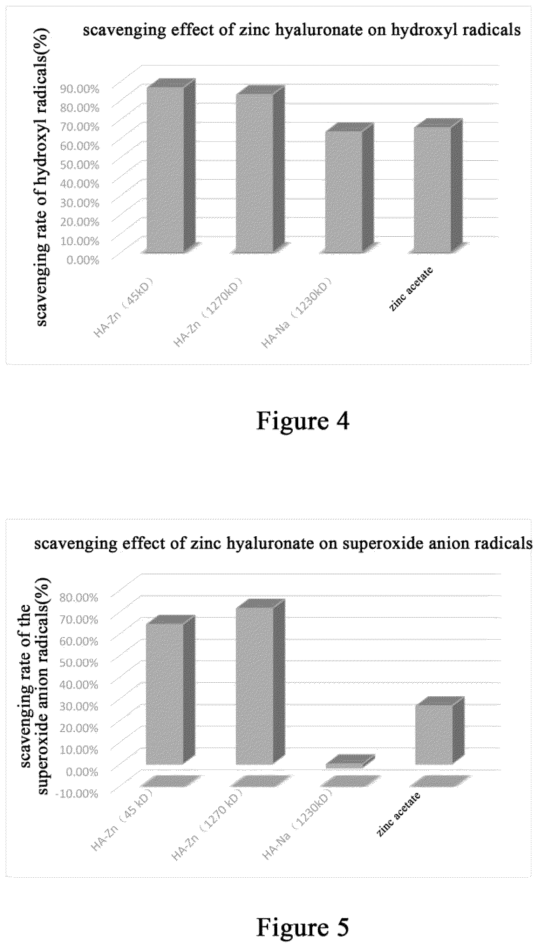

The results in Figure 4 show that HA-Zn has a significant effect on the scavenging of hydroxyl free radicals, and the hydroxyl free radical scavenging rate of the zinc hyaluronate with low molecular weight is higher, reaching 85%; at the same concentration, HA-Na and zinc acetate dihydrate alone also have a certain ability to scavenging hydroxyl free radicals, but the scavenging rate is significantly lower than that of HA-Zn. Therefore, zinc hyaluronate has a significant effect in scavenging hydroxyl free radicals, reflecting that zinc hyaluronate has a good antioxidant effect and is of great significance for maintaining normal physiological activities and anti-aging of the body.

(2) Superoxide anion scavenging effect

Experimental Materials:

0.5% zinc hyaluronate I (Example 9, 1270 kD) solution sample, 0.5% zinc hyaluronate II (Example 10, 45 kD) sample; 0.5% sodium hyaluronate solution sample (Huaxi Biotechnology Co., Ltd., 1230 kD), and 0.5% zinc acetate (dihydrate) solution.

Experimental methods:

The pyrogallol self-oxidation method was used. Under weakly alkaline conditions, pyrogallol can undergo self-oxidation reaction to generate superoxide anions and a colored intermediate product with a characteristic absorption peak at 320 nm. In the initial trial stage, the amount of intermediate product is linearly related to time. Since the self-oxidation rate depends on the concentration of superoxide anions, when a superoxide anion scavenger is added, it can react quickly with superoxide anions to inhibit the self-oxidation reaction, thereby preventing the accumulation of intermediates and weakening the light absorption of the solution at 320 nm. Therefore, the scavenging effect of the scavenger on superoxide anions can be evaluated by measuring the A320 value.
The scavenging rate is calculated as follows:

$$\text{scavenging rate of superoxide anion free radical(\%)} = (A_0-A_x)/A_0\times100\%$$

As shown in Figure 5, HA-Zn has a significant effect on the scavenging of superoxide anion free radicals, among which hyaluronic acid zinc with high molecular weight has a higher scavenging ability, with a scavenging rate of more than 70%. Although zinc acetate has a certain ability to scavenge superoxide anion free radicals, it is significantly lower than that of HA-Zn, and HA-Na has almost no ability to scavenge superoxide anion free radicals. Therefore, zinc hyaluronate has a significant effect in scavenging superoxide anion free radicals, which reflects that zinc hyaluronate has a good antioxidant effect.

Test Example 2.6 Reduction of scar formation

Experimental materials: zinc hyaluronate sample (Example 9, 1270 kD)

Experimental methods:

Based on the differences in amino acids in the three peptide chains that make up tropocollagen, collagen can be divided into four types: I, II, III, and IV. Normal skin collagen is mainly composed of types I and III, and the ratio of the two is about 3.5:1.

During scar formation, the absolute value and relative proportion of type II collagen fibers show a decreasing trend, while type I collagen fibers show an increasing trend. Type I collagen is a coarse fiber and is the material basis of scar tissue fibrosis; type II collagen is a fine fiber and is the main component of reticular fibers. As scarring forms, type II fibers are gradually replaced by thick type I fibers, destroying the reticular structure of normal skin. The biological characteristics of normal skin change accordingly, with the type I/III collagen ratio dropping significantly, the collagen fibers becoming disordered, and the fibers becoming thick, thus showing the hallmark appearance of scarring.

The fibroblasts were treated with TGF-$\beta$1 to establish an in vitro scar model. The scar model was treated with 0.05 mg/ml zinc hyaluronate test substance working solution, and images were collected using a fluorescence microscope within 48 hours. The image magnification was 400 times (eyepiece 10$\times$, objective lens 40$\times$). The scar repair efficacy was evaluated by observing the changes in the contents of Collagen I and Collagen III in fibroblasts. (This test was conducted by Guangdong Biocell Biotechnology Co., Ltd.)

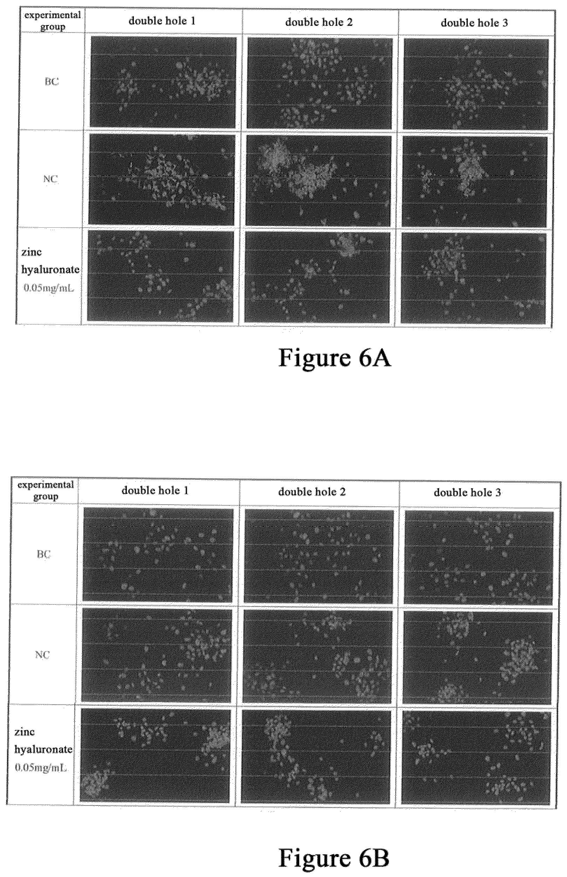

BC was the positive control and NC was the negative (blank) control. As shown in Figures 6A and 6B, compared with the NC group, after the sample was treated with zinc hyaluronate at a concentration of 0.05 mg/mL for 24 hours, the content of Collagen I was extremely significantly decreased (P<0.05); the content of Collagen III was decreased, but there was no significant difference.

In summary, in the in vitro culture system, the sample zinc hyaluronate at a concentration of 0.05 mg/mL can significantly inhibit the synthesis of Collagen I, thereby affecting the expression ratio of Collagen I and Collagen III proteins and inhibiting the occurrence of scars caused by excessive expression of Collagen I.

**Claims**

1. A method for preparing zinc hyaluronate, comprising the following steps:
adding sodium hyaluronate to an acidic aqueous organic medium comprising a zinc salt to perform one replacement to obtain a zinc hyaluronate precipitate.

2. The method according to claim 1, wherein the mass ratio of the added amount of the sodium hyaluronate to the zinc salt is 1:0.5 to 1:3.5, preferably 1:1 to 1:3.

3. The method according to claim 1 or 2, wherein the concentration of an organic solvent in the acidic aqueous organic medium comprising a zinc salt is 55% to 95% (v/v), preferably 55% to 70% (v/v), preferably the concentration of zinc ion is 1% to 3% (w/v), and preferably the pH of the acidic aqueous organic medium comprising a zinc salt is 5.0 to 6.9.

4. The method according to any one of claims 1 to 3, wherein the molecular weight of the sodium hyaluronate is 1 kDa to 3000 kDa.

5. The method according to any one of claims 1 to 4, wherein replacement time is 1 to 24 hours.

6. The method according to any one of claims 1 to 5, further comprising washing the zinc hyaluronate precipitate with a washing solution, then dehydrating the washed precipitate with a dehydrating solution, and drying the precipitate to obtain a zinc hyaluronate powder.

7. A zinc hyaluronate with low molecular weight prepared by the method according to any one of claims 1 to 6, preferably, the molecular weight of the zinc hyaluronate with low molecular weight is less than or equal to 1000 kDa.

8. A zinc hyaluronate with high molecular weight prepared by the method according to any one of claims 1 to 6, preferably, the molecular weight of the zinc hyaluronate with high molecular weight is greater than 1000 kDa.

9. A zinc hyaluronate, wherein the molecular weight of the zinc hyaluronate is 2 kDa to 2500 kDa, and the transmittance of the zinc hyaluronate in a 0.5wt% aqueous solution is $\geq$ 88.1%, preferably $\geq$ 99.1%.

10. Use of the zinc hyaluronate prepared by the method according to any one of claims 1 to 8 or the zinc hyaluronate according to claim 9 in skin moisturizing, skin oil control, inhibiting harmful bacteria on the skin, anti-oxidation, scar repair, inhibiting scar , and preventing skin wound infection;

preferably, the use in inhibiting harmful bacteria on the skin comprises a use in removing acne and dandruff;
preferably, the use in preventing skin wound infection comprises preventing skin wound infection caused by intradermal injection and microneedle injection.

11. A method for skin moisturizing, skin oil control, inhibiting harmful bacteria on the skin, anti-oxidation, scar repair, inhibiting scar or preventing skin wound infection, comprising administering the zinc hyaluronate prepared by the method according to any one of claims 1 to 8 or the zinc hyaluronate according to claim 9 to a subject,

preferably, the inhibiting harmful bacteria on the skin comprises removing acne and dandruff,
preferably, the preventing skin wound infection comprises preventing skin wound infection caused by intradermal injection and microneedle injection.

12. Use of the zinc hyaluronate prepared by the method according to any one of claims 1 to 8 or the zinc hyaluronate according to claim 9 in skin care products.

13. Use of zinc hyaluronate in reducing skin oil content, preferably, the zinc hyaluronate is a zinc hyaluronate with low molecular weight,
further preferably, the molecular weight of the zinc hyaluronate with low molecular weight is 2 kDa to 1000 kDa, preferably 5 kDa to 500 kDa.

14. Use of zinc hyaluronate in reducing porphyrin content in skin oil, preferably, the zinc hyaluronate is a zinc hyaluronate with low molecular weight,
further preferably, the molecular weight of the zinc hyaluronate with low molecular weight is 2 kDa to 1000 kDa, preferably 5 kDa to 500 kDa.

15. Use of zinc hyaluronate in removing hydroxyl radicals from the skin surface, preferably, the zinc hyaluronate is a zinc hyaluronate with low molecular weight,
further preferably, the molecular weight of the zinc hyaluronate with low molecular weight is 2 kDa to 1000 kDa, preferably 5 kDa to 500 kDa.

16. Use of zinc hyaluronate in removing superoxide anion free radicals from the skin surface, preferably, the zinc hyaluronate is a zinc hyaluronate with high molecular weight,
further preferably, the molecular weight of the zinc hyaluronate with high molecular weight is 1000 kDa to 2500 kDa, preferably 1100 kDa to 2000 kDa.

effect of zinc hyaluronate Ⅰ on the water content in the stratum corneum

|  | T0 | T1W | T2W | T4W |
|---|---|---|---|---|
| zinc hyaluronate | 100.00 | 155.07 | 136.36 | 132.96 |
| blank | 100.00 | 149.61 | 129.79 | 129.05 |

## Figure 1A

effect of zinc hyaluronate Ⅱ yaon the water content in the stratum corneum

|  | T0 | T1W | T2W | T4W |
|---|---|---|---|---|
| zinc hyaluronate | 100.00 | 143.76 | 133.22 | 137.18 |
| blank | 100.00 | 136.15 | 124.68 | 131.46 |

## Figure 1B

effect of zinc hyaluronate I on skin oil content

| | T0 | T1W | T2W | T4W |
|---|---|---|---|---|
| zinc hyaluronate | 0.00 | 49.56 | 46.75 | 38.80 |
| blank | 0.00 | 51.78 | 48.25 | 44.50 |

Figure 2A

effect of zinc hyaluronate II on skin oil content

| | T0 | T1W | T2W | T4W |
|---|---|---|---|---|
| zinc hyaluronate | 0.00 | 60.00 | 56.50 | 67.10 |
| blank | 0.00 | 73.11 | 63.33 | 76.00 |

Figure 2B

**effect of zinc hyaluronate I on the porphyrin content in the T region**

| | T0 | T1W | T2W | T4W |
|---|---|---|---|---|
| zinc hyaluronate | 100.00 | 117.30 | 99.11 | 90.70 |
| blank | 100.00 | 109.26 | 105.97 | 95.18 |

## Figure 3A

**effect of zinc hyaluronate II on the porphyrin content in the T region**

| | T0 | T1W | T2W | T4W |
|---|---|---|---|---|
| zinc hyaluronate | 100.00 | 109.06 | 90.48 | 93.58 |
| blank | 100.00 | 121.44 | 104.70 | 100.24 |

## Figure 3B

Figure 4

Figure 5

| experimental group | double hole 1 | double hole 2 | double hole 3 |
|---|---|---|---|
| BC | | | |
| NC | | | |
| zinc hyaluronate 0.05mg/mL | | | |

## Figure 6A

| experimental group | double hole 1 | double hole 2 | double hole 3 |
|---|---|---|---|
| BC | | | |
| NC | | | |
| zinc hyaluronate 0.05mg/mL | | | |

## Figure 6B

<table>
<tr><td colspan="2">**INTERNATIONAL SEARCH REPORT**</td><td colspan="2">International application No.<br>**PCT/CN2023/089192**</td></tr>
</table>

**A. CLASSIFICATION OF SUBJECT MATTER**

C08B37/08(2006.01)i; A61K8/73(2006.01)i

According to International Patent Classification (IPC) or to both national classification and IPC

**B. FIELDS SEARCHED**

Minimum documentation searched (classification system followed by classification symbols)

IPC分类: C08B A61K

Documentation searched other than minimum documentation to the extent that such documents are included in the fields searched

Electronic data base consulted during the international search (name of data base and, where practicable, search terms used)

CNABS, CNTXT, CNKI, ENTXT, VEN, ISI Web of Knowledge: 玻璃酸钠, 玻璃酸锌, 透明质酸钠, 透明质酸锌, 置换, 化妆品, 控油, 油脂, 卟啉, 清除, 自由基, 抗老, hyaluronate, sodium, zinc, hyaluronic acid, replace, precipitate, cosmetics, radical, eliminate, scavenge, oil, control

**C. DOCUMENTS CONSIDERED TO BE RELEVANT**

| Category* | Citation of document, with indication, where appropriate, of the relevant passages | Relevant to claim No. |
|---|---|---|
| X | CN 111647100 A (SHANDONG BLOOMAGE HYINC BIOPHARM CO., LTD.) 11 September 2020 (2020-09-11)<br>description, paragraphs 2, 9-21, and 33 | 1-16 |
| X | CN 1760214 A (SHANDONG FURUIDA PHARMACEUTICAL GROUP CO., LTD.) 19 April 2006 (2006-04-19)<br>Claims | 1-6, 9 |
| A | US 2009215719 A1 (Q P CORP.) 27 August 2009 (2009-08-27)<br>entire document | 1-16 |
| A | JP 2016166133 A (Q P CORP.) 15 September 2016 (2016-09-15)<br>entire document | 1-16 |
| A | WO 2006121210 A1 (TAKATA SEIYAKU CO., LTD.; SERIZAWA, ISAO; GOTO, HIDEKI; ONO, HAJIME; MIYAZAKI, HIROYUKI) 16 November 2006 (2006-11-16)<br>entire document | 1-16 |

☐ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **18 August 2023** | **20 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/089192**

| Patent document cited in search report | | | Publication date (day/month/year) | Patent family member(s) | | | Publication date (day/month/year) |
|---|---|---|---|---|---|---|---|
| CN | 111647100 | A | 11 September 2020 | CN | 111647100 | B | 01 March 2022 |
| CN | 1760214 | A | 19 April 2006 | CN | 100355790 | C | 19 December 2007 |
| US | 2009215719 | A1 | 27 August 2009 | KR | 20140063898 | A | 27 May 2014 |
| | | | | KR | 20080002848 | A | 04 January 2008 |
| | | | | EP | 2463309 | A1 | 13 June 2012 |
| | | | | EP | 2463309 | B1 | 12 June 2013 |
| | | | | JP | 2006265287 | A | 05 October 2006 |
| | | | | JP | 4576583 | B2 | 10 November 2010 |
| | | | | WO | 2006101030 | A1 | 28 September 2006 |
| | | | | EP | 1865002 | A1 | 12 December 2007 |
| | | | | EP | 1865002 | A4 | 30 March 2011 |
| | | | | EP | 1865002 | B1 | 16 May 2012 |
| | | | | US | 2015166685 | A1 | 18 June 2015 |
| | | | | KR | 20110108425 | A | 05 October 2011 |
| | | | | KR | 101420485 | B1 | 16 July 2014 |
| | | | | US | 8933054 | B2 | 13 January 2015 |
| | | | | JP | 2009256683 | A | 05 November 2009 |
| | | | | JP | 5130264 | B2 | 30 January 2013 |
| | | | | CN | 101146830 | A | 19 March 2008 |
| | | | | CN | 101146830 | B | 16 November 2011 |
| | | | | CN | 102174122 | A | 07 September 2011 |
| | | | | CN | 102174122 | B | 07 August 2013 |
| JP | 2016166133 | A | 15 September 2016 | None | | | |
| WO | 2006121210 | A1 | 16 November 2006 | JPWO | 2006121210 | A1 | 18 December 2008 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 100355790 C
- CN 111647100 A
- CN 102834417 A
- CN 112691049 A
- CN 102961396 A